# EUROPEAN PATENT SPECIFICATION

(11) **EP 3 165 229 B1**
(45) Date of publication and mention of the grant of the patent: **13.02.2019**
(21) Application number: 16197205.4
(22) Date of filing: 04.11.2016
(51) Int. Cl.: A61K 36/185, A61K 36/39, A61P 35/00, A61P 15/00, A61P 43/00, A61K 31/047, A61K 31/19, A61K 31/56, A61K 31/366

(54) **COMPOSITION FOR THE PREVENTION OR TREATMENT OF POLYCYSTIC OVARY SYNDROME AND ITS RELATED SYMPTOMS**
ZUSAMMENSETZUNG ZUR PRÄVENTION ODER BEHANDLUNG DES POLYZYSTISCHEN OVARIALSYNDROMS UND VERWANDTER SYMPTOME
COMPOSITION POUR LA PRÉVENTION OU LE TRAITEMENT DU SYNDROME D'OVAIRE POLYKYSTIQUE ET SES SYMPTÔMES ASSOCIÉS

(30) Priority: 05.11.2015 IT UB20155087
(43) Date of publication of application: 10.05.2017
(73) Proprietor: Pharmamol S.R.L., 86100 Campobasso (IT)
(72) Inventor: Scapagnini, Giovanni, 95124 Catania (IT); Di Marco, Roberto Maria Antonio, 95123 Catania (IT); Mangano, Katia, 95123 Catania (IT); Longo Sormani, Sonia, 22040 Alzate Brianza (IT)
(74) Representative: Coppo, Alessandro

(56) References cited:
- US-A1- 2006 198 907
- US-B1- 8 349 373
- NORDIO M ET AL: "The combined therapy with myo-inositol and D-chiro-inositol reduces the risk of metabolic disease in PCOS overweight patients compared to myo-inositol supplementation alone", RIVISTA EUROPEA PERLE SCIENZE MEDICHE E FARMACOLOGICHE // EUROPEAN REVIEW FOR MEDICAL AND PHARMACOLOGICAL SCIENCES // REVUE EUROPEENNE POUR LES SCIENCES MEDICALES ETPHARMACOLOGIQUES, ROME, IT, vol. 16, no. 5, 1 May 2012 (2012-05-01), pages 575-581, XP009168775, ISSN: 0392-291X
- TANQUILUT N C ET AL: "Hypoglycemic effect of Lagerstroemia speciosa (L.) Pers. on alloxan-induced diabetic mice", JOURNAL OF MEDICINAL PLANT RESEARCH, ACADEMIC JOURNALS, NG, vol. 3, no. 12, 1 December 2009 (2009-12-01), pages 1066-1071, XP009189657, ISSN: 1996-0875
- DATABASE WPI Week 201278 Thomson Scientific, London, GB; AN 2012-N91796 XP002756710, & CN 102 631 516 A (MA S) 15 August 2012 (2012-08-15)
- MOHANRAJ REMYA ET AL: "Sweet Potato (Ipomoea batatas [L.] Lam) - A Valuable Medicinal Food: A Review", JOURNAL OF MEDICINAL FOOD, MARY ANN LIEBERT, LARCHMONT, NY, US, vol. 17, no. 7, 1 July 2014 (2014-07-01), pages 733-741, XP009189668, ISSN: 1096-620X
- NOWAK D A ET AL: "The effect of flaxseed supplementation on hormonal levels associated with polycystic ovarian syndrome: A case study", CURRENT TOPICS IN NUTRACEUTICAL RESEARCH 200711 US, vol. 5, no. 4, November 2007 (2007-11), pages 177-181, XP009189672, ISSN: 1540-7535

## Description

### FIELD OF THE INVENTION

The present invention relates to a composition for use in the prevention or treatment of polycystic ovary syndrome and its related symptoms.

The present invention originates in the field of dietetic products, herbal medicines and food supplements.

In particular, the present invention relates to a herbal product for oral use intended for the prevention or treatment of polycystic ovary syndrome and its related symptoms.

### PRIOR ART

Polycystic ovary syndrome is a complex syndrome which is characterized, from the anatomopathological point of view, by the presence of enlarged and micropolycystic ovaries and, from the clinical point of view, by a number of endocrine-metabolic changes.

Polycystic ovary syndrome, also known as PCOS, is one of the most common disorders in women of childbearing potential, with a prevalence rate ranging between 6 and 10% of the female population.

PCOS generally manifests around the time of puberty and presents with symptoms such as menstrual disorders, hirsutism and obesity. Endocrine disorders typically observed in this syndrome are often associated with metabolic disorders, whose manifestations become more and more evident with increasing age, until reaching prevailing conditions after menopause.

PCOS is largely spread and currently represents one of the most frequent causes for infertility in the Western female population.

PCOS has a multifactorial etiopathogenesis. A condition of hyperandrogenism is usually observed, which triggers an excessive acyclic estrone production that, in its turn, determines a gonadotropin hyperproduction, mainly LH. The imbalance in LH/FSH ratio reduces follicular maturation, resulting in anovulation and hyperstimulation of theca cells, with androgen hyperproduction.

Concomitantly, in the presence of PCOS, a reduced conversion of androgens into oestrogens at level of granulosa cells occurs, determining an androgen accumulation which induces follicular maturation regression or chronic follicular atresia.

Therefore PCOS is the result of a number of different pathogenetic mechanisms which tend to feed off each other, thus determining the onset and worsening of symptoms.

From the anatomopathological point of view, polycystic ovary appears enlarged, smooth or mammillated, with an increased cortical thickness. At subcapsular level, several ovarian cysts are present, with a diameter ranging from 4 to 7 mm, with a reduced number of granulosa cells and hyperplasia of theca cells. Cysts consist of antral follicles, blocked at their development stage, which did not undergo atresia. The presence of ovarian cysts (polycystic ovary) does not necessarily imply a polycystic ovary syndrome, hence polycystic ovary and polycystic ovary syndrome are to be considered different entities.

The most frequent PCOS-related endocrine disorders include in 80% of cases menstrual irregularities, such as oligomenorrhoea, amenorrhoea, metrorrhagia, infertility; in 60% of cases hyperandrogenism, which commonly manifests itself with hirsutism, acne, alopecia; and in 50% of cases obesity.

The most frequent metabolic disorders include glucose intolerance, diabetes mellitus, fibrinolytic defects with hyperfibrinogenemia, dyslipidaemias, hypertension, with a consequent increase in cardiovascular risks.

Menstrual disorders are present in almost all the subjects suffering from PCOS, leading to amenorrhoea in 50-60% of cases whilst functional bleeding is observed in 25% of patients and infertility in 70-85% of cases.

Hyperandrogenism commonly manifests itself as hirsutism. In subjects with PCOS also seborrhoea and acne are frequently observed, the former related to an excessive stimulation of sebaceous glands due to high androgen concentrations, the latter induced by occlusion of pores in the skin, associated with skin inflammation and possible formation of pus. Moreover, the changes in the composition of sebum produced by sebaceous glands lead to hyperkeratosis of the excretory duct, determining congestion phenomena with formation of comedones.

Furthermore, the action on the glandular secretion induced by esterases produced by the microorganism Corynebacterium acnes, determines a perifocal inflammation of the surrounding tissues.

In some cases, PCOS hyperandrogenism can subsequently lead to alopecia. PCOS often manifests itself as obesity, frequently associated with a condition of hyperinsulinism related to insulin-resistance. It has been observed that insulin-resistance plays a key role in the genesis of this syndrome in 33% of lean women with PCOS. The most frequently reported insulin-resistance-related metabolic disorders include changes in the lipid profile, reduced glucose tolerance or type-2 diabetes mellitus, hyperfibrinogemia and fibrinolytic defects, arterial hypertension. Currently, PCOS preventive measures are adopted in the presence of risk factors such as prepubertal and/or early pubertal hypertrichosis and overweight. These measures include a restriction of the calorie intake from the diet and an increased physical activity.

The pharmacological therapy is reserved to the most serious cases or when a regularization of the menstrual cycle should be obtained. Typically, the pharmacological therapy involves the administration of one or more hormonal substances.

A first type of therapy is represented by the oestroprogestinic therapy, scheduling the association of oestrogens with a progestin possessing antiandrogenic properties, in order to regulate the menstrual cycle and reduce hyperandrogenism signs.

However, as with all hormonal therapies, oestroprogestinic therapy is not devoid of inconveniences related to its use.

Furthermore, if the patient is trying to become pregnant, ovulation should be induced by discontinuation of oestrogen/progestin drugs or by administration of clomiphene, an oestrogen with weak activity acting also as anti-oestrogen. The use of this drug can however lead to important undesirable effects, such as hot flushes, changes in some blood components and visual disturbances, also of significant nature.

As an alternative, ovulation induction may be performed by administering gonadotropins, typically FSH, a hormone acting on follicles in the last step of their maturation process. However, FSH use may lead to superovulation phenomena. In addition, excessive FSH doses may cause multiple ovulations and the ovarian hyperstimulation syndrome, a condition characterized by an increased vascular permeability, with fluid transfer to the extracellular compartment and consequent hypovolaemia, haemoconcentration and concomitantly ascites, pleural and pericardial effusions.

A therapeutic alternative to the hormonal treatment is represented by the hypoglycaemic therapy, involving the administration of oral hypoglycaemic agents able to reduce the correlations between hyperinsulinaemia and hormonal-metabolic changes. Also this type of therapy has some limitations, besides exposing the patient to the risk of a therapy that, being a long-term treatment, can definitely impair the pancreatic function.

The article "The combined therapy with myo-inositol and D-chiro-inositol reduces the risk of metabolic disease in PCOS overweight patients compared to myo-inositol supplementation alone" to Nordio M. et al. published in Rivista Europea Per le Scienze Mediche e Farmacologiche // European Review for Medical and Pharmacological Sciences vol. 16, no. 5, 1 May 2012 (2012-05-01) pages 575-581 discloses that the combined administration of myo-inositol (MI) and D-chiro inositol (DCA) may be considered as the first line approach treatment in PCOS overweight patients, who need to control insulin levels and increase ovarian MI content, reducing the risk of developing a metabolic disease.

The patent US 8 349 373 B1 of 8 January 2013 discloses a dietary supplement for use in weight loss program, which alleviates insulin resistance in the presence of the metabolic syndrome, including in combination banaba leaf extract inositol and berberine.

The patent application US 2006/198907 of 7 September 2006 discloses the pharmaceutical, therapeutic and dietary compositions derived from leaf of the *Lagerstroemia speciosa* plant. Polycistic ovarian disease (PCO) is among the glucose metabolism disorders that the disclosed compositions are useful for preventing or treating.

Therefore, currently there is a need for treatments that are able to prevent or treat initial forms of PCOS, with a major efficacy with respect to simple diets but also deprived of the side effects commonly observed with the pharmacological therapies presently used.

It is one of the aims of the present invention to provide a composition capable of preventing or treating PCOS-related symptoms, which is substantially deprived of the side effects commonly observed with the pharmacologically active substances currently used to manage this syndrome.

Another aim of the present invention is to provide a composition suitable to treat or prevent PCOS which is based on active substances of plant origin which, being deprived of significant side effects, allow the long-term treatment of this syndrome. A further aim of the present invention is to provide a treatment method to prevent or treat PCOS and/or its related symptoms by administering active substances of plant origin or derivation which, being deprived of significant side effects, allow the long-term treatment of this syndrome.

### SUMMARY OF THE INVENTION

The present invention originates from the finding that by combining and administering a plant extract of Banaba (*Lagerstroemia speciosa L*.) with inositol and Ipomoea Batatas, a number of signals and secondary messenger molecules playing a significant role in certain biological processes are activated, including insulin signal transduction, thus determining a beneficial synergistic effect on the ovulatory function and PCOS-related symptoms.

According to a first aspect thereof, a composition for use as claimed in the appended claim 1 is provided.

Further embodiments of the composition for the use of the invention are indicated in the herewith appended claims 2-9.

The inventors have discovered that by combining a Banaba extract, inositol in one of its biologically active forms and Ipomoea Batatas, a synergistic sensitizing effect of the cell membrane on insulin is produced, which decreases insulin resistance thereby determining an increase in the ovarian frequency and a reduction in hyperandrogenism, without inducing the onset of side effects typically related to the hormonal therapy.

The inventors have also found that the synergistic effect obtained by the combination of the aforementioned three active substances of the composition is further enhanced by adding lignans obtained, for example, from a flaxseed dry extract.

Consequently, according to some embodiments, the composition for use of the invention further comprises lignans or a lignan-containing extract.

Typically, the components with synergistic activity which are present according to one of the embodiments of the composition for use of the invention, are provided in a pharmaceutically or nutritionally effective amount.

The composition of the invention finds application in the prevention or treatment of polycystic ovary syndrome and/or as adjuvant for symptoms associated with this condition.

According to some embodiments, the composition for use of the invention is a pharmaceutical composition, a herbal product, a supplement or dietetic product which can be introduced in the diet of a female subject suffering from polycystic ovary syndrome.

According to some aspects of the invention, a preparation or kit is provided, containing a plant extract of Banaba, inositol or its biologically active salts or isomers, Ipomoea Batatas and optionally lignans, as a combined preparation for simultaneous, separate or subsequent use in the prophylaxis or treatment of polycystic ovary syndrome.

According to the present invention it is provided a composition comprising a plant extract of Banaba, inositol and Ipomoea Batatas and an edible and/or physiologically acceptable carrier for use in the prevention or treatment of polycystic ovary syndrome.

According to a further aspect, the present disclosure provides a method for the prevention or treatment of polycystic ovary syndrome comprising the administration to a female subject of a composition based on a plant extract of Banaba, inositol and Ipomoea Batatas and optionally lignans, in an edible and/or physiologically acceptable carrier.

The present invention will now be described in detail with reference to the following enclosed figures.

### BRIEF DESCRIPTION OF THE DRAWINGS

Some features and advantages of the present invention will be made clear by the attached drawings wherein:
Figure 1 shows the effect of the oral administration of Dcin (inositol), Lag (Banaba or *Lagerstroemia speciosa* extract), Ipo (Ipomoea Batatas extract), taken individually or as the combination Dcin+Lag+lpo (50 mg/45 days), on the glucose tolerance profile in rats with letrozole-induced PCOS, n=10;
Figure 2 shows the in-vivo effect of Dcin, Lag, Ipo, taken individually or as the combination Dcin+Lag+lpo, on ovarian steroidogenic enzyme activity in letrozole-induced rat models, n=10.

### DETAILED DESCRIPTION OF THE INVENTION

According to some aspects of the invention, the inventors have found that by combining a plant extract of Banaba (*Lagerstroemia speciosa L.*), with inositol or its salts or isomeric forms, and Ipomoea Batatas, an increased cell sensitivity to insulin is produced, able to compensate, at least partially, for insulin resistance and hyperinsulinaemia, conditions which play an important role in the pathogenesis and/or onset of PCOS and its related symptoms.

The present invention originates from the finding that the combined use of three active components/extracts - each having, as target, biological mechanisms that contribute to determine polycycstic ovary syndrome - develops a synergistic effect of prevention and reduction of symptoms related to PCOS in female subjects.

According to a first aspect, the present invention relates to a composition for use in preventing and/or treating polycystic ovary syndrome, comprising a synergistic combination of a plant extract of Banaba (*Lagerstroemia speciosa L*.), inositol or its salts or isomeric forms, and Ipomoea Batatas - each of these three components being provided in an effective amount - as well as an edible or physiologically acceptable carrier.

A bioactive ingredient or component of the composition for use of the invention is a vegetable extract or a vegetable part of Banaba plant, or a biologically active component contained in a Banaba extract, in particular corosolic acid and/or ellagitannins.

Banaba, or *Lagerstroemia speciosa (L.)* is a semi-deciduous tree native to the Far East which grows wild in India, Southeast Asia, the Philippines and Malaysia. Within the context of the invention it is possible to use vegetable portions of Banaba *(Lagerstroemia speciosa),* a plant extract of Banaba, specifically Banaba leaf extract and/or an active component obtained from Banaba, including corosolic acid [(2-alpha,3-beta)-2,3-dihydroxy-urs-12-en-28-oic acid, pentacyclic triterpenic compound, C₃₀H₄₈O₄ p.m. 472.69) and/or ellagitannins, typically selected among lagerstroemine, flosine B, reginine A and mixtures thereof [Hayashi et al. 2002].

In particular, corosolic acid is suitable as it activates cellular glucose uptake, as demonstrated on Ehrlich ascites tumour cells [Murakami et al. 1993]. Furthermore, glucose uptake is stimulated also by ellagitannins, as demonstrated on rat epididymal adipocytes [Hayashi et al. 2002].

These mechanisms have been identified as the main responsible for the glucose-lowering activity of Banaba plant extracts.

According to some embodiments, the composition for use of the invention contains corosolic acid and one or more ellagitannins.

Corosolic acid and/or ellagitannins are biologically active compounds that can be obtained by extraction, for example dry extraction, from Banaba plant, in particular from its leaves.

In some embodiments, a suitable plant extract of Banaba is a dry extract obtained, for example, by grinding and drying of a portion of the plant, particularly leaves.

Typically, the extraction of corosolic acid and/or ellagitannins from a portion of Banaba plant can take place by traditional techniques, for example by extraction with a suitable solvent from plant portions, as indicated by Bohlman and Zdero in Phytochemistry 1982; 21:2543-49.

In some embodiments, the composition for use of the invention contains an extract of Banaba or its biologically active compounds corosolic acid and/or ellagitannins, in an amount ranging from 0.001 to 30% by weight, from 0.01 to 15% by weight or from 0.1 to 10% by weight.

In some embodiments, the composition contains a vegetable portion or an extract of Banaba in an amount ranging from 10 to 2000 mg, from 50 to 1000mg, from 100 to 500mg.

According to some embodiments, the posology used ranges from 10 to 300 mg/day of Banaba dry extract, for example from 32 to 48 mg/day of dry extract.

Typically, a portion of vegetable tissue of Banaba or a plant extract from a vegetable portion of Banaba, for example leaves, exerts a glucose-lowering action. There are studies documenting the glucose-lowering action of Banaba extracts, such as the study of Ikeda et al. 1999 performed in patients with type II diabetes treated with placebo or with Banaba extract three times daily. The study points out a significant decrease in glucose levels in patients treated with Banaba extract.

In another more recent study [Judy et al. 2003], patients with moderate type II diabetes have been treated for two weeks with placebo or with 32 or 48 mg of extract. A significant dose-dependent reduction in blood glucose levels was observed also in this study in patients treated with Banaba, which was not the case for patients treated with placebo.

The glycaemic control induced by Banaba extracts is considered similar to that exerted by insulin, occurring through an increase in the activity of glucose transporters across the cell membrane [De Tommasi et al. 1991, Liu et al. 2001]. However, unlike insulin, Banaba extracts do not induce adipocyte differentiation but rather inhibit it in a dose-dependent manner [Liu et al. 2001]. This inhibition is consequent to the reduction in PPARgamma2 (peroxisome proliferator-activated receptor gamma 2) and transport protein 4 (GLUT4) in the cells [Liu et al. 2001]. The glucose-lowering property and the absence of adipogenic effects have been experimentally confirmed in mice [Kakuda et al. 1996, Suzuki et al. 1999], and would make Banaba extracts particularly useful in type 2 diabetes of moderate entity. In genetically obese mice (Type II, KK-Ay), Banaba extracts do not exert particular effects on blood lipid levels, but inhibit lipid accumulation in the liver by 65% [Suzuki et al. 1999].

A further Banaba action was also reported in terms of lipid accumulation and distribution. In particular, in genetically obese rats it was observed that corosolic acid inhibits adipose tissue lipoprotein lipase, and this action could explain the anti-fat effect of Banaba extracts observed in experimental animals [Yoshikawa et al. 2002].

Banaba leaf extracts of the composition for use of the invention exert an insulin-like effect and control blood glucose levels in subjects predisposed to diabetes or with type II diabetes of moderate entity. However, they do not produce a fat accumulation in adipocytes, as happens with insulin.

These activities developed by Banaba and its extracts are appreciable, as hyperinsulinism, frequently observed in subjects with PCOS, contributes to the onset of hyperandrogenism. In fact, insulin acts on the ovary synergistically with LH, thus determining an increase in androgen concentration to which a significant portion of PCOS symptoms is attributable.

In addition, insulin contributes to hyperandrogenism also through other mechanisms such as i) stimulation of the hypothalamic pulse generator, inducing an increase in LH synthesis; ii) stimulation of theca cells; iii) reduction in SHBG, which determines an increase in androgen amount; iv) maintenance of obesity and adipose tissue where the conversion of androgens to oestrogens occurs; v) enhancement of ACTH action on adrenal glands, with increase in adrenal androgen synthesis.

This leads to a vicious circle that reinforces polycystic ovary syndrome. Consequently, insulin resistance does not develop at ovarian level. Furthermore, in 40% of subjects with PCOS, high prolactin blood levels are observed, as a consequence of increased circulating estrone levels, which may contribute to menstrual irregularities.

Typically, Banaba extracts are substantially devoid of toxicity and significant undesirable effects.

Another component of the composition for use of the invention is inositol.

In the context of the present invention, the term "inositol" refers to the available forms of inositol, such as its physiologically acceptable salts and inositol isomers, such as D-chiro-inositol or myo-inositol and mixtures thereof.

In particular, inositol is naturally available as phytic acid in some types of plant fibers, and as myo-inositol in the meat.

Inositol, in its different forms, increases insulin action by improving cell sensitivity to insulin.

In some embodiments, the composition contains inositol in one or more of its isomeric forms or salts, in an amount ranging from 0.001 to 30% by weight, from 0.01 to 15% by weight or from 0.1 to 10% by weight.

The inventors have found that by combining a Banaba extract, or the biologically active principles obtained from Banaba extraction, with inositol in one of its forms, a synergistic effect of cell sensitization to insulin and up-regulating activity on insulin receptors (InsR) is obtained.

This mechanism is of particular value in the treatment of polycystic ovary syndrome, as the up-regulation of insulin receptors, with their associated increased number on the cell membrane, enhances membrane sensitivity to insulin; therefore glucose can more easily and largely permeate the cell membrane, thus inducing the body to reduce the amount of circulating insulin and decreasing considerably the risks of hyperinsulinaemia, which is related to polycystic ovary syndrome progression.

The inventors have further found that the combined effect of Banaba extract and inositol is surprisingly enhanced by the presence of Ipomoea Batatas in the composition.

In particular, a ternary composition based on inositol, Banaba extract and Ipomoea Batatas shows a synergistic effect inducing a reduction in polycystic ovary syndrome typical symptoms.

A component of the composition for use of the invention comprises a portion or an extract of Ipomoea Batatas, commonly referred to as "white sweet potato". According to some embodiments, in the composition an extract or a portion of Ipomoea Batatas L. tuber is used.

According to some embodiments, the tuber comes from the variety Caiapo, native to South America and commonly used in Japan.

In some embodiments, the composition comprises a dry extract of Ipomoea Batatas.

By way of example, a suitable dry extract of Ipomoea Batatas can be obtained by an extraction method including the following steps
- Crushing/grinding of Ipomoea Batatas tuber,
- Extraction with a physiologically acceptable solvent, such as ethanol, to be performed, for example, three times for a period of time ranging from 30 minutes to 2 hours for each extraction, typically for a period of one hour,
- Filtration,
- Filtrate concentration, for example under vacuum with concentration eluent,
- Spray drying and optionally
- Grinding, sieving or screening of the resulting extract.

In some embodiments, the composition for use of the present invention contains an extract or portion of Ipomoea Batatas in an amount ranging from 0.1 to 1000 mg, from 1 to 500mg, from 50 to 300mg.

In some embodiments, the extract or portion of Ipomoea Batatas is present in an amount ranging from 0.001 to 20% by weight, from 0.01 to 30% by weight, from 1 to 10% by weight of the composition.

In particular, it was observed that by combining a portion or extract of Ipomoea Batatas tuber with inositol, extract of Banaba or its biologically active components therein contained, according to one of the aforementioned embodiments, a synergistic effect leading to cell sensitization to insulin is obtained, able to improve symptoms related to polycystic ovary syndrome.

This synergism of action of the aforementioned active components is further enhanced by the presence of lignans in the formulation of the composition for the use of the invention.

Lignans are natural products deriving from plants, typically the flax plant. Suitable lignans are obtained by extraction from flax plant seeds.

Within the context of the present invention, the term "lignans" refers to cinnamic acid dimers containing a dibenzylbutane skeleton, and in particular they comprise phenylpropanoid dimer compounds with two C₆C₃ units linked by a β,β' bond, as defined in "LG-0 and LG-1 Introduction and Patent Structures" - IUPAC www.chem.qmul.ac.uk/iupac/lignan/LG0n1.html, whose content is deemed to be referred to in full herein.

According to some embodiments, lignans are contained or extracted from flax plant seeds (linum usitatissimum L.).

In some embodiments, a suitable lignan or flaxseed extract is sesoisolariciresinol diglucoside, referred to in literature as SDG, which is typically present in flax plant seeds.

In general, suitable lignans are the ones described by Schmidt TJ et al. in "Lignans in seeds of Linum species" Phytochemistry 2012 Oct., 82:89-99, as an example of aryldihydronaphthalene-type lignans, such as arylnaphthalene lignan justicidin B, and aryl tetralin-type lignans, also in the form of esters with aliphatic carboxylic acids such as 6-methoxypodophyllotoxin-7-O-n-hexanoate.

In some embodiments, the composition for use of the present invention contains lignans extracted from the flax plant. The extraction of lignans from flax seeds can be carried out using conventional techniques as described, for example, by Essam F. Al-Jumaily et al. in "Extraction and Purification of lignan compound from flax seed Linum usitatissimum" Asian Journal of Plant Science and Research, 2012, 2(3):306-312, the content of which is by this reference incorporated herein in full. In some embodiments, the flaxseed extract is an extract obtained using traditional techniques, for example by extraction with a suitable solvent from plant portions, as indicated, for example, by Bohlman and Zdero in Phytochemestry 1982; 21 :2543-49.

In the formulation for the use of the invention, lignans or flaxseed extracts exert an antioxidant action contributing to the improvement in PCOS symptoms.

In some embodiments, the composition for use of the present invention contains lignans, in particular flax plant extracts, in an amount ranging from 1 to 1500mg, from 10 to 800mg, from 100 to 400mg.

In some embodiments, the composition for use of the present invention contains lignans in an amount ranging from 0.001 to 10% by weight, from 0.01 to 5% by weight or from 0.1 to 1% with respect to the total weight.

In the context of the invention, the term "synergism or synergistic activity" means an activity that is greater than the sum of the activities of the active ingredient when taken alone. Typically, synergism occurs when at least two biologically active substances or ingredients interact in ways that enhance or highlight one or more of their effects. Therefore, two active substances or ingredients evidently producing similar effects, will sometimes produce exaggerated or reduced effects when used concomitantly, being thus a quantitative assessment necessary to distinguish these cases from a simple additive action (Tallarida RJ. Drug Synergism: its detection and applications. J PharmacolExpTher. 2001 Sep:298(3):865-72).

In some embodiments, the composition for use of the present invention comprises further active substances or ingredients.

In some embodiments, the composition for use of the present invention further comprises one or more vitamins, for example B-complex vitamins, niacin, vitamin A, vitamin C, vitamin PP, being B-complex vitamins the preferred ones. B-complex vitamins of choice are vitamin B6, vitamin B12 and mixtures thereof.

In some embodiments, the composition of the present invention further comprises folacin or folic acid.

According to some embodiments, the composition for the use of the present invention further comprises one or more micronutrients and/or minerals such as salts of Mg, K, Na, Zn, Fe, Cr, Se, Mn and others.

The term "carrier" as used herein indicates a means, excipient, diluent with which the association of therapeutic or active ingredients is administered.

Any carrier and/or excipient suitable for the preparation form desired for administration to human beings can be used with the compounds described in the present invention.

For the purposes of the present application, the term "physiologically acceptable" indicates edible substances which are approved by the health authorities for use in pharmaceutical, nutritional or food applications.

Within the context of the present application, by the term "combination" it is meant that one or more active ingredients are added to or mixed with one or other ingredients to yield the formulation of the invention. The term "combination" should therefore not be understood in the sense that the active ingredients combine with one another with the formation of chemical bonds.

A physiologically acceptable carrier can be a pharmaceutically acceptable carrier. The term "edible" means a physiologically acceptable solvent that can be assimilated by the human body.

The compositions for the use of the present invention comprise any composition produced by administering the association of active ingredients of the present invention and a physiologically or pharmacologically acceptable carrier. Said compositions are suited to food, nutritional, pharmaceutical or dietary use in mammals, in particular in human beings.

According to some embodiments, the composition for the use of the invention is a dietary supplement.

The composition for use of the invention can take a wide variety of preparation forms, according to the desired administration route.

For example, for oral administration, the composition can be in solid form, for example capsule, tablet, powder, granule or formulations for prolonged release of the active ingredients. The compositions in solid form, in particular capsules containing a mixture of the ingredients according to any of the above described embodiments, are particularly suitable.

The preparations in solid form can comprise one or more carriers, for example starches, sugars, microcrystalline cellulose, and optionally diluents, granulation agents, lubricants, ligands and disintegrating agents.

The tablets, pills, capsules and granulates can also contain a ligand such as tragacanth, acacia, maize starch or gelatin; excipients such as dicalcium phosphate; a disintegrating agent such as maize starch, potato starch, alginic acid; a lubricant such as magnesium stearate; a sweetening agent such as sucrose, lactose or saccharin. If desired, the tablets can be coated by means of traditional techniques.

When the unitary pharmaceutical form is a capsule, it can contain, in addition to the materials of the above-mentioned type, a liquid carrier such as a fatty oil.

In the case of preparations in liquid form for oral administration, as in the case of suspensions, emulsions and solutions, a suitable carrier can be chosen among water, glycols, oils, alcohol and mixtures thereof.

Flavourings, preservatives, colourings and similar may also be present in the composition.

In some embodiments, the vegetable extracts or active ingredients contained in the composition for the use of the present invention can be combined or mixed as active ingredients in intimate mixture with a suitable edible carrier and/or an excipient according to techniques of the pharmaceutical and food industry or traditional nutrition techniques.

The compositions for pharmaceutical or nutritional use can be adequately presented in a single pharmaceutical form and prepared for example by mixing the ingredients of the formulation, by means of any one of the methods well known in pharmaceutical or food technology.

In some embodiments, the composition for the use of the invention further comprises one or more additional components such as additives, fillers, stabilizers, emulsifiers, texturizers, film-forming agents, plasticizers, wetting agents and thickeners. Various other materials can be present as coatings or to modify the physical form of the pharmaceutical unit. For example, tablets can be coated with shellac, sugar or both. To prevent breakdown as it passes through the upper part of the gastrointestinal tract, the composition can be a formulation with enteric coating.

A syrup or elisir can contain, in addition to the active ingredient, sucrose as a sweetening agent, methyl and propylparabens as preservatives, a colouring agent and a flavouring agent such as cherry or orange flavour.

The compositions for the use of the invention are prepared according to conventional pharmaceutical manufacturing techniques, for example by mixing the active ingredients with suitable excipients and/or physiologically acceptable carriers to obtain the desired pharmaceutical form.

In some embodiments, in the compositions for the use of the present invention the active ingredients are normally formulated in dosage units. The dosage unit can contain from 0.1 to 1000 mg of active ingredient or vegetable extract containing the active ingredient per dosage unit for the daily administration.

In some embodiments of the composition for the use of the invention, the dosage unit is a sachet which can contain from 1 to 50 g, or from 5 to 30g of composition.

According to some embodiments, the formulation will contain amounts of active ingredients which will depend on severity of polycystic ovary syndrome and its related symptoms, condition, any further treatment in progress, the individual's health and the response to the association of active ingredients. In some embodiments, the dose ranges from 0.001% by weight to about 60% by weight of the formulation.

According to some embodiments, the composition for the use of the invention is a medicine.

According to some embodiments, the composition for the use of the invention is a supplement, a nutritional product or a herbal product.

The following examples are provided mainly to illustrate the present invention and are not intended to limit the protective field as resulting from the attached claims.

### EXAMPLE 1

| Raw materials | mg/cap |
|---|---|
| D-Chiro Inositol | 100.0 |
| Flaxseed D.E. standardized to 20% Lignans | 80.00 |
| Ipomoea Batatas L. | 80.00 |
| Lagerstroemia speciosa-Banaba standard. to 1% corosolic acid | 80.00 |
| Zinc gluconate | 50.48 |
| Vitamin B6-hydrochloride | 4.392 |
| Folic acid | 0.515 |
| Vitamin B12 standardized to 0.1% | 4.500 |
| Micronized silica | 5.000 |
| Parmcompress | 37.10 |
| Magnesium stearate | 5.000 |
| Talc | 5.000 |

### EXAMPLE 2

Composition for the treatment of PCOS and its related symptoms having the following formulation

| | | Improves insulin activity | Improves glycaemic control | Improves circulating levels of hormones |
|---|---|---|---|---|
| D-chiro-inositol | 250 mg | +++ | | + |
| Lignans (from flax) | 200 mg | | | +++ |
| | | | | |
| Ipomoea Batatas L. | 100 mg | | +++ | |
| Lagerstroemia speciosa L. | 250 mg | | +++ | |
| Zn | 22.5 mg | + | | |
| vitamin B6 | 3 mg | | | |
| Folacin | 400 mcg | | | |
| vitamin B12 | 3.75 mcg | | | |

| | | | | |
|---|---|---|---|---|
| For each single ingredient of the formulation the type of performed activity is indicated, which is quantified on a scale from one to three indicated by the symbol +. | | | | |

### EXAMPLE 3

### Experimental trials

Letrozole, a non-steroidal aromatase inhibitor inducing androgen excess and promoting the development of polycystic ovary syndrome or PCOS, was used in order to establish a PCOS rat model. Animals with PCOS exhibit insulin resistance and hyperinsulinaemia, leading to an increased plasma glucose concentration, as observed in rats following oral glucose tolerance tests (OGTT).

The animals were divided into 6 groups of 10 animals each.

Five groups were treated with letrozole to induce the development of PCOS, whilst one group was used as negative control.

Three out of the five groups were treated by administering d-chiro-inositol, Banaba extract and Ipomoea Batata extract taken alone, whilst one group received a synergistic composition containing d-chiro-inositol, Banaba extract and Ipomoea Batata extract.

Specifically, the animals were treated for 30 days by oral route with DCIN (d-chiro inositol), Lag (Banaba or Lagerstroemia speciosa extract) and Ipo (Ipomoea Batatas extract), administered alone or as the combination DCIN + Lag + Ipo (50 mg / 45 days).

Oral glucose tolerance tests (OGTT) were performed in all the groups of rats after 30 days of treatment.

Subsequently ovarian steroid enzyme activities in the ovarian tissue of the mitochondrial fraction were examined.

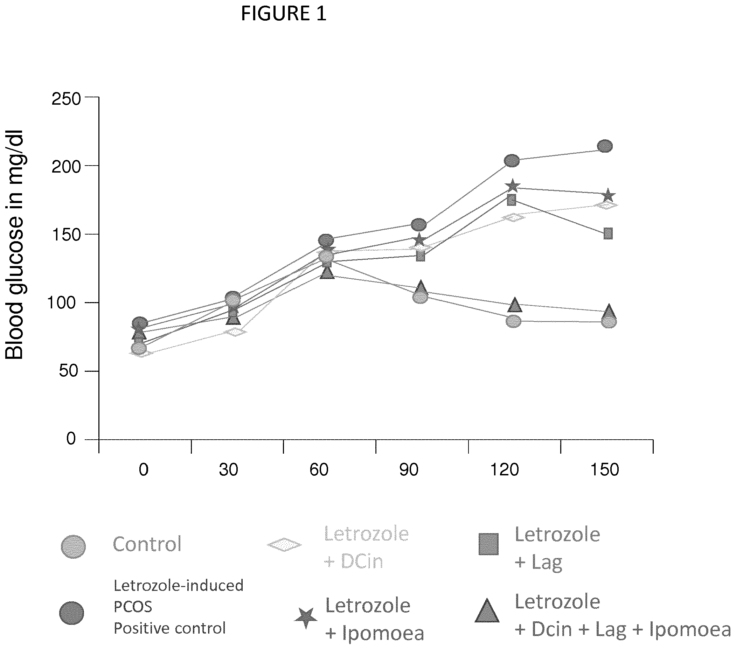

The graph in Figure 1 shows a significant reduction in glucose tolerance in rats with PCOS with respect to the control group; PCOS rats treated with each of the active substances taken alone showed a slight improvement in glucose tolerance. Surprisingly, rats with PCOS treated with the preparation containing the combined three active substances showed a restored glucose tolerance, comparable to that of the control group.

Figure 1 highlights how letrozole-induced PCOS positive rats developed changes in steroid enzyme activity and an impaired androgen production related to the ovarian function, showing a major activity for 3 beta-dehydrogenase (HSD) and 17 beta-HSD with respect to the control groups.

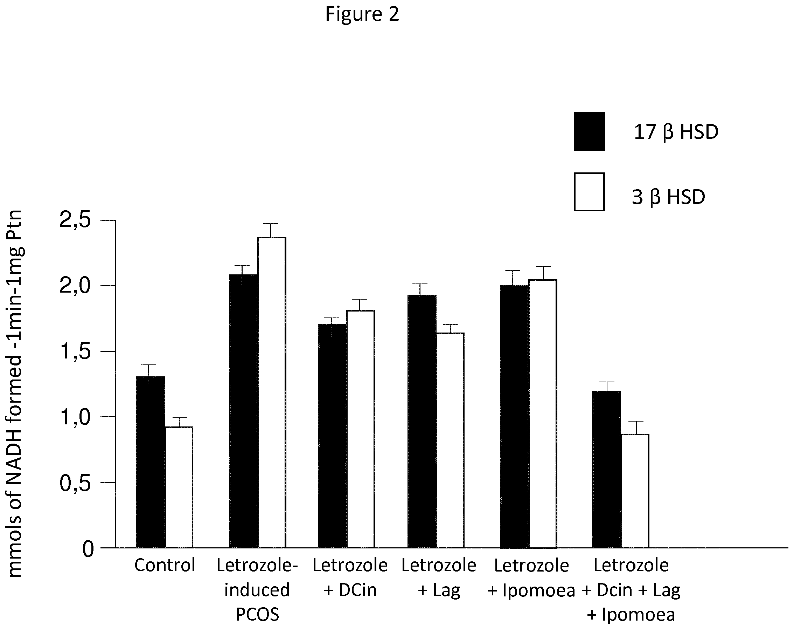

This condition is indicative of a higher androgen production in rats with polycystic ovary syndrome (PCOS). A high androgen level induces insulin-resistance through the activation of the lipolytic cascade and changes in muscle histology. Letrozole-induced PCOS rats treated with the three active substances taken alone showed a moderate improvement in terms of androgen production (Figure 2).

This study demonstrates the efficacy of the composition containing the combination of the three active substances in restoring the activities of these enzymes to control levels, thus confirming a synergistic effect of this three active substance-containing composition in its ability to modulate the activities of these enzymes.

The composition is able to decrease 3βHSD activity while maintaining 17β HSD activity at physiological levels, thus reducing total androgen production and increasing steroidogenesis flow towards oestrogen synthesis.

## Claims

1. Composition comprising i) corosolic acid, ellagitannins and/or an extract of Banaba (*Lagerstroemia speciosa),* ii) inositol and/or its salts and/or isomeric forms, iii) Ipomoea Batatas and a physiologically acceptable carrier, for use in the prevention and/or treatment of polycystic ovary syndrome.

2. Composition for use according to claim 1, **characterized in that** it comprises an extract of Banaba (*Lagerstroemia speciosa),* D-chiro-Inositol, an extract or portion of Ipomoea Batatas and a physiologically acceptable carrier.

3. Composition for use according to claim 1 or 2, **characterized in that** corosolic acid and ellagitannins are contained in a vegetable extract of Banaba (*Lagerstroemia speciosa),* preferably a dry Banaba leaf extract.

4. Composition for use according to claim 1 or 2, **characterized in that** it further comprises lignans.

5. Composition for use according to claim 4 wherein lignans are extracted from flax plant seeds.

6. Composition for use according to any one of claims 1-5 further comprising at least one additional biologically active component selected from micronutrients, mineral salts, enzymes, folacin and vitamins, in particular vitamins B6 and/or B12.

7. Composition for use according to any one of claims from 1 to 6, **characterized in that** it is a food supplement, a dietetic product, a medicine, a herbal product or a nutritional supplement.

8. Composition for use according to any one of claims 1-7, **characterized in that** it is in solid form, preferably a capsule.

9. Composition for use according to claim 1 in solid form for oral administration, preferably in the pharmaceutical form of capsules.

## Patentansprüche

1. Zusammensetzung, aufweisend
i) Korosolsäure, Ellagitannine und / oder ein Extrakt von Banaba (Lagerstroemia speciosa),
ii) Inositol und / oder dessen Salze und / oder isomere Formen,
iii) Ipomoea Batatas und einen physiologisch akzeptablen Trägerstoff,
zur Verwendung bei der Prävention und / oder Behandlung des polyzystischen Ovarialsyndroms.

2. Zusammensetzung zur Verwendung gemäß Anspruch 1,
**dadurch gekennzeichnet, dass** sie ein Extrakt von Banaba (Lagerstroemia speciosa), D-Chiro-Inositol, einen Extrakt oder einen Teil von Ipomoea Batatas und einen physiologisch akzeptablen Trägerstoff aufweist.

3. Zusammensetzung zur Verwendung gemäß Anspruch 1 oder 2,
**dadurch gekennzeichnet, dass** Korosolsäure und Ellagitannine in einem Pflanzenextrakt von Banaba (Lagerstroemia speciosa) enthalten sind, vorzugsweise ein trockener Banaba-Blätterextrakt.

4. Zusammensetzung zur Verwendung gemäß Anspruch 1 oder 2,
**dadurch gekennzeichnet, dass** diese weiter Lignane aufweist.

5. Zusammensetzung zur Verwendung gemäß Anspruch 4, wobei Lignane aus Samen der Flachspflanze extrahiert werden.

6. Zusammensetzung zur Verwendung gemäß einem der Ansprüche 1 bis 5, weiter aufweisend zumindest eine zusätzliche biologisch aktive Komponente, ausgewählt aus Spurenelementen, Mineralsalzen, Enzymen, Folsäure und Vitaminen, insbesondere den Vitaminen B6 und oder B12.

7. Zusammensetzung zur Verwendung gemäß einem der Ansprüche 1 bis 6,
**dadurch gekennzeichnet, dass** sie ein Nahrungsmittelzusatz, ein diätisches Produkt, eine Medizin, ein pflanzliches Mittel oder ein Nahrungsergänzungsmittel ist.

8. Zusammensetzung zur Verwendung gemäß einem der Ansprüche 1 bis 7,
**dadurch gekennzeichnet, dass** sie in fester Form, vorzugsweise als Kapsel, vorliegt.

9. Zusammensetzung zur Verwendung gemäß Anspruch 1 in fester Form zur oralen Gabe, vorzugsweise in pharmazeutischer Form von Kapseln.

## Revendications

1. Composition comprenant i) de l'acide corosolique, des ellagitannins et/ou un extrait de Banaba (*Lagerstroemia speciosa*), ii) de l'inositol et/ou ses sels et/ou ses formes isomériques, iii) Ipomoea Batatas et un véhicule physiologiquement acceptable, pour une utilisation dans la prévention et/ou le traitement du syndrome de l'ovaire polykystique.

2. Composition pour une utilisation selon la revendication 1, **caractérisée en ce qu'**elle comprend un extrait de Banaba (*Lagerstroemia speciosa*), du D-chiro-inositol, un extrait ou une portion d'Ipomoea Batatas et un véhicule physiologiquement acceptable.

3. Composition pour une utilisation selon la revendication 1 ou 2, **caractérisée en ce que** de l'acide corosolique et des ellagitannins sont contenus dans un extrait végétal de Banaba (*Lagerstroemia speciosa*), de préférence un extrait sec de feuille de Banaba.

4. Composition pour une utilisation selon la revendication 1 ou 2, **caractérisée en ce qu'**elle comprend en outre des lignanes.

5. Composition pour une utilisation selon la revendication 4, dans laquelle des lignanes sont extraits de graines de lin.

6. Composition pour une utilisation selon l'une quelconque des revendications 1 à 5 comprenant en outre au moins un composant biologiquement actif supplémentaire sélectionné parmi des micronutriments, des sels minéraux, des enzymes, de la folacine et des vitamines, en particulier des vitamines B6 et/ou B12.

7. Composition pour une utilisation selon l'une quelconque des revendications 1 à 6, **caractérisée en ce qu'**elle est un complément alimentaire, un produit diététique, un médicament, un produit de phytothérapie ou un complément nutritionnel.

8. Composition pour une utilisation selon l'une quelconque des revendications 1 à 7, **caractérisée en ce qu'**elle est sous forme solide, de préférence une capsule.

9. Composition pour une utilisation selon la revendication 1 sous forme solide pour une administration orale, de préférence sous la forme pharmaceutique de capsules.
